# EUROPÄISCHE PATENTSCHRIFT

(11) **EP 4 021 523 B1**
(45) Veröffentlichungstag und Bekanntmachung des Hinweises auf die Patenterteilung: **06.03.2024**
(21) Anmeldenummer: 20764073.1
(22) Anmeldetag: 26.08.2020
(51) Int. Cl.: A61L 31/14, A61L 31/16, A61B 50/30, A61L 31/04

(54) **POLYMERFOLIE UND POLYMERTASCHE ZUR AUFNAHME EINES ZU IMPLANTIERENDEN MEDIZINISCH-TECHNISCHEN PRODUKTS**
POLYMER FILM AND POLYMERIC BAG FOR HOLDING A MEDICAL-TECHNICAL PRODUCT TO BE IMPLANTED
FILM POLYMÈRE ET POCHE POLYMÈRE DESTINÉS À RECEVOIR UN PRODUIT MÉDICO-TECHNIQUE À IMPLANTER

(30) Priorität: 30.08.2019 DE 102019213178
(43) Veröffentlichungstag der Anmeldung: 06.07.2022
(73) Patentinhaber: Resorba Medical GmbH, 90475 Nürnberg (DE)
(72) Erfinder: AHLERS, Michael, 90547 Stein (DE); BAUER, Markus, 90461 Nürnberg (DE); RUHL, Beate, 34286 Spangenberg (DE)
(74) Vertreter: Rau, Schneck & Hübner Patentanwälte Rechtsanwälte PartGmbB
(86) Internationale Anmeldenummer: PCT/EP2020/073884
(87) Internationale Veröffentlichungsnummer: WO 2021/037932

(56) Entgegenhaltungen:
- WO-A1-2016/159885
- US-A1- 2008 128 315

## Beschreibung

Die vorliegende Patentanmeldung nimmt die Priorität der deutschen Patentanmeldung DE 10 2019 213 178.6 in Anspruch.

Die Erfindung betrifft eine Polymerfolie zur Einbettung eines in einen menschlichen Organismus zu implantierenden medizinisch-technischen Produkts. Außerdem betrifft die Erfindung eine aus mindestens einer solchen Polymerfolie hergestellte Polymertasche zur Aufnahme eines solchen medizinisch-technischen Produkts.

Bei einem derartigen zu implantierenden medizinisch-technischen Produkt kann es sich beispielsweise um Knie- und Hüft-Endoprothesen oder um einen Herzschrittmacher handeln. Jährlich werden in den USA und in Europe mehrere Hunderttausend solcher Produkte implantiert. In den meisten Fällen funktioniert dies reibungslos. Es gibt aber eine steigende Anzahl von Problemfällen, bei denen es zu Komplikationen kommt, insbesondere weil es am Ort der Implantation zu einer Infektion kommt. Solche Komplikationen können für den betroffenen Patienten sehr gravierende Folgen bis hin zum Tod haben. Außerdem ist die Bekämpfung der Komplikationen mit erheblichen Kosten verbunden.

Um Infektionen bei der Implantation solcher medizinisch-technischer Produkte zu vermeiden, werden u.a. Antibiotika-haltige Schwämme und Folien eingesetzt, wie z.B. die von der Anmelderin unter der Marke "GENTA-Coll" vertriebenen dementsprechenden Produkte.

Eine weitere Methode zur Infektionsvermeidung wird in der US 9 023 114 B2 und in der US 2008/0128315 A1 beschrieben. Demnach wird das betreffende medizinisch-technische Produkt vor seiner Implantation in einer Tasche platziert, welche aus einem resorbierbaren Polymer-Material besteht, das zusätzlich mit einem infektionshemmenden Wirkstoff versehen ist. Allerdings sind diese Taschen teilweise spröde und steif und damit schwierig in der Handhabung. Außerdem kann ihre Verträglichkeit bedingt durch die Verwendung synthetischer Komponenten eingeschränkt sein.

Eine ähnliche Tasche wird in der WO 2014/046 741 A1 beschrieben. Diese Tasche besteht aus einer extrazellulären Matrix, wobei es sich um ein gereinigtes Stück eines tierischen Gewebes handelt. Diese Matrix kann mit einer Lösung eines infektionshemmenden Wirkstoffs getränkt werden. Allerdings ist die dann resultierende Haftung des Wirkstoffs auf der Matrix gering. Er kann insbesondere auch wieder abgespült werden. Außerdem verbraucht er sich nach dem Einsetzten der Tasche in den Patientenkörper relativ schnell, so dass die infektionshemmende Wirkung nur vergleichsweise kurze Zeit gegeben sein kann.

Weiterhin wird in dem Fachartikel von Chen, D. W. et al., "Sustainable release of vancomycin, gentamicin and lidocaine from novel electrospun sandwich-structured PLGA/collagen nanofibrous membranes", International Journal of Pharmaceutics 430 (2012), Seiten 335-341 eine zur Einbettung in den menschlichen Körper bestimmte Membran beschrieben, die einen Sandwich-Aufbau aus drei mittels Elektrospinning hergestellten und damit jeweils aus Nanofasern bestehenden Teilschichten hat. Die beiden äußeren Schichten setzen sich aus einer Kombination der Materialien Polylactid-Polyglycolid (PLGA) und Kollagen zusammen, wohingegen die zentrale Schicht PLGA und die drei Wirkstoffe Vancomycin, Gentamicin und Lidocain enthält. Aufgrund der Herstellung mittels Elektrospinning und der daraus resultierenden Zusammensetzung aus Nanofasern ist diese Membran nicht dicht. Sie hat feine Poren.

In der WO 2016/159 885 A1 wird eine weitere antibiotische Abdeckung zur Aufnahme eines zu implantierenden medizinisch-technischen Produkts beschrieben. Diese Abdeckung umfasst eine Folie aus mindestens einer Polymerschicht enthaltend ein biodegradierbares elastomeres Polymermaterial, mindestens ein in der Polymerschicht dispergiertes Antibiotikum und mindestens eine Öffnung zum Einführen des medizinisch-technischen Produkts. Die Folie weist Löcher auf. Außerdem kann die Abdeckung das medizinisch-technische Produkt nur teilweise aufnehmen.

Eine Aufgabe der Erfindung besteht nun darin, eine Polymerfolie der eingangs bezeichneten Art mit gegenüber dem Stand der Technik verbesserten Eigenschaften anzugeben.

Zur Lösung dieser Aufgabe wird eine Polymerfolie entsprechend den Merkmalen des Patentanspruchs 1 angegeben. Die erfindungsgemäße Polymerfolie weist als Hauptbestandteil ein biologisch abbaubares und vom menschlichen Körper resorbierbares Polymer natürlichen Ursprungs, nämlich ein Atelokollagen, auf und hat einen Polymergehalt. Außerdem weist sie als weitere Bestandteile mindestens zwei antimikrobielle Wirkstoffe mit jeweils unterschiedlichem Wirkmechanismus auf. Die Polymerfolie hat insofern einen Wirkstoffgesamtgehalt. Das Verhältnis des Wirkstoffgesamtgehalts zu dem Polymergehalt beträgt mindestens 15 %, wobei der Polymergehalt aber größer als der Wirkstoffgesamtgehalt ist. Darüber hinaus ist die Polymerfolie biegbar und modulierbar, so dass die Polymerfolie an das medizinisch-technische Produkt eng angelegt werden kann, wobei sich die Polymerfolie zumindest weitestgehend an die Konturen des medizinisch-technischen Produkts anpasst. Weiterhin ist die Polymerfolie ununterbrochen. Außerdem bildet das Polymer eine Polymermatrix, in die die mindestens zwei antimikrobiellen Wirkstoffe homogen verteilt eingebettet sind. Darüber hinaus hat die Polymerfolie für jeden der mindestens zwei antimikrobiellen Wirkstoffe jeweils einen Wirkstoffeinzelgehalt, wobei die Wirkstoffeinzelgehalte um höchstens 20 % voneinander abweichen.

Die erfindungsgemäße Polymerfolie hat einen außergewöhnlich hohen Wirkstoffgesamtgehalt von mindestens 15 % und insbesondere von mindestens 20% bezogen auf den Polymergehalt. Dadurch und auch, weil sich die antimikrobiellen Wirkstoffe in ihrem jeweiligen Wirkmechanismus voneinander unterscheiden, ist die Infektionshemmung und/oder Keimunterdrückung besonders effizient. Jeder antimikrobielle Wirkstoff hat einen anderen Wirkmechanismus, so dass er gegen eine andere Keimklasse seine Wirkung entfalten kann. Durch den hohen Wirkstoffgesamtgehalt und die unterschiedlichen Wirkmechanismen werden praktisch alle Keime, die sich ggf. an dem zu implantierenden Gerät (=medizinisch-technischen Produkt) festsetzen oder bilden könnten, abgetötet und damit neutralisiert. Folglich werden Infektionen am Ort der Implantation sicher unterdrückt. Außerdem wird durch die Verwendung von mindestens zwei antibiotischen Wirkstoffen mit jeweils unterschiedlichem Wirkmechanismus die bestehende Resistenz mancher Keime gegenüber einzelnen Wirkstoffen umgangen. Infektionen werden trotzdem verhindert, da mindestens einer der Wirkstoffe trotz der möglichen singulären Resistenz eines Keims noch wirksam ist.

Die durch die Polymerfolie, in die das zu implantierende Gerät eingebettet ist, an dem Ort der Implantation eingebrachte antimikrobielle Wirkstoffkonzentration ist um ein Vielfaches höher, als sich dies durch eine systemische Gabe von antimikrobiellen Wirkstoffen erzielen lässt. Die antimikrobiellen Wirkstoffe werden durch die zur Einbettung verwendete Polymerfolie vorteilhafterweise direkt an dem Ort, an dem sie benötigt werden, platziert, und dies in sehr hoher Konzentration. Da die Wirkstoffplatzierung nur am Implantationsort erfolgt, ist die Gesamtbelastung des Patienten trotz der lokal sehr hohen Wirkstoffkonzentration über den gesamten Organismus betrachtet relativ niedrig und vor allem deutlich niedriger als bei einer oralen oder parenteralen Gabe. Insofern wird ein Beitrag zu Bekämpfung der zunehmenden Antibiotika-Resistenz von Keimen geleistet, ohne dabei das Patientenwohl zu gefährden. Die lokale Keimunterdrückung ist sehr hoch und effizient.

Die mindestens zwei antimikrobiellen Wirkstoffe befinden sich insbesondere auch innerhalb des Folienmaterials. Sie stehen also vorzugsweise während der gesamten Dauer des Abbaus des Polymers zur Verfügung. Dadurch bleibt die infektionshemmende Wirkung der Polymerfolie über einen langen Zeitraum erhalten.

Insbesondere hat die Polymerfolie genau zwei antimikrobielle Wirkstoffe. Aber auch drei oder vier antimikrobielle Wirkstoffe sind möglich. Vorzugsweise sind höchstens vier antimikrobielle Wirkstoffe enthalten.

Das Polymer natürlichen Ursprungs ist aber trotzdem der Hauptbestandteil der erfindungsgemäßen Polymerfolie, wobei das Polymer natürlichen Ursprungs - wie auch jedes andere Polymer natürlichen Ursprungs - insbesondere einen inhärenten Anteil an Feuchtigkeit aufweist. Dieser Feuchteanteil (oder Feuchtegehalt) liegt beispielsweise zwischen 3% und 20%, vorzugsweise zwischen 5% und 18%. Er ist Bestandteil des Polymergehalts, welcher hier u.a. zur Spezifizierung der Polymerfolie verwendet wird. Dieser Polymergehalt ist größer als der (hohe) Wirkstoffgesamtgehalt, welcher sich insbesondere aus der Summe der Wirkstoffeinzelgehalte jedes der mindestens zwei antimikrobiellen Wirkstoffe zusammensetzt.

Trotz des sehr hohen Wirkstoffgesamtgehalts lässt sich die Polymerfolie sehr gut handhaben und verarbeiten. Dies liegt insbesondere an der Biegbarkeit und vorzugsweise auch an der Modulierbarkeit der Polymerfolie. Letzteres bedeutet, dass die Polymerfolie an ein Anlageobjekt mit weitgehend beliebiger Konturen, wie eines der einzubettenden medizinisch-technischen Produkte, eng angelegt werden kann, wobei sich die Folie aufgrund ihrer Biegbarkeit und Geschmeidigkeit komplett oder zumindest weitestgehend an die Konturen des Anlageobjekts anpasst bzw. diesen Konturen folgt. Sie kann sich diesen weitgehend beliebigen Konturen des Anlageobjekts praktisch vollständig anschmiegen. Dies ist auch deshalb von besonderer Bedeutung, da vergleichbare bestehende Produkte eben gerade nicht biegbar und insbesondere auch nicht modulierbar sind. Bei zu starker Verbiegung können bestehende Vergleichsprodukte sogar brechen. Sehr hohe Wirkstoffgehalte können zur bereichsweisen Kristallisation führen, was zu einer Brüchigkeit führen kann. Bei der erfindungsgemäßen Polymerfolie kommt es aufgrund der guten Verträglichkeit des resorbierbaren Polymers natürlichen Ursprungs trotz des höheren Wirkstoffgehalts zu keiner Kristallisation. Sie ist daher sehr geschmeidig. Die erfindungsgemäße Polymerfolie hat vorteilhafterweise keine Beschränkung hinsichtlich des Grads einer möglichen Verbiegung.

Letzteres verbessert die Handhabung und Verarbeitbarkeit. Außerdem wird der Einsatz bei Operationen erleichtert. So können über das zu implantierende Produkt überstehende Bereiche der Polymerfolie umgebogen werden, so dass die Größe der Einheit aus dem zu implantierenden Produkt und der zu dessen Einbettung verwendeten Polymerfolie im Wesentlichen durch das zu implantierende Produkt bestimmt ist. Insofern muss die Größe des Schnitts zur Öffnung des Patientenkörpers an der Implantationsstelle auch nur an der Größe des zu implantierenden Produkts bemessen werden. Die zusätzlich vorhandene Polymerfolie führt aufgrund ihrer Biegbarkeit zu keiner wesentlichen Vergrößerung der vom Arzt einzubringenden Schnittöffnung. Bei Einbettungsprodukten des Standes der Technik, die keine vergleichbare Biegbarkeit aufweisen, bestimmt dagegen die Größe des zur Einbettung verwendeten Einbettungsprodukts anstelle der Größe des zu implantierenden Produkts die Größe der vom Arzt einzubringenden Schnittöffnung, welche dann größer ausfällt. Dadurch wird der Patient stärker belastet. Demgegenüber entlastet die erfindungsgemäße Polymerfolie den Patienten insofern, als eine kleinere Schnittöffnung für die Implantation ausreicht.

Außerdem erleichtert die Biegbarkeit auch die Einbettung des medizinisch-technischen Produkts in die erfindungsgemäße Polymerfolie. So kann das medizinisch-technische Produkt z.B. in gängiger Weise mit der Polymerfolie eingewickelt oder umwickelt werden. Das dafür erforderliche ggf. auch mehrfache Umklappen erlaubt die Polymerfolie ohne weiteres. Die Einbettung kann bevorzugt sehr eng und dicht erfolgen.

Eine weitere Entlastung des Patienten resultiert bei der erfindungsgemäßen Polymerfolie dadurch, dass sie aus oder mit einem biologisch abbaubaren und vom menschlichen Körper resorbierbaren Polymer natürlichen Ursprungs hergestellt ist. Ein solches insbesondere hoch gereinigtes Polymer natürlichen Ursprungs lässt sich vom menschlichen Körper leichter abbauen und resorbieren als die synthetischen Polymere, die bei den Produkten des Standes der Technik zum Einsatz kommen. Auch ist ein solches Polymer besser verträglich als ein Produkt aus Gewebestücken. Die erfindungsgemäße Polymerfolie hat insofern eine höhere Biokompatibilität als der Stand der Technik bei zugleich besserer Keimunterdrückung. Das resorbierbare Polymer natürlichen Ursprungs ist vorzugsweise ein insbesondere vollständig rekonstituiertes Material. Es ist ein Atelokollagen. Ein solches Polymer weist eine besonders hohe Biokompatibilität auf. Der menschliche Körper kann es besonders gut abbauen bzw. resorbieren.

Bei dem einzubettenden und zu implantierenden medizinisch-technischen Produkt kann es sich vorzugsweise um einen Herzschrittmacher, einen implantierbaren Defibrillator, ein Gerät zur Resynchronisationstherapie, eine implantierbare Medikamentenpumpe, ein Neurostimulationsimplantat (Spinal Cord Stimulator, SCS), ein Kunstherz, ein Blutdruck-regulierendes Implantat, einen Neuromodulatoren (Deep Brain Stimulation Device) oder auch um mechanische Implantate, Prothesen, usw. handeln. Zur Einbettung wird das betreffende medizinisch-technische Produkt insbesondere mit der erfindungsgemäßen Polymerfolie umgeben, vorzugsweise möglichst vollständig. Frei bleiben insbesondere nur etwaige (elektrische) Zu- oder Ableitungen und deren Anschlussstellen, wie sie beispielsweise bei Herzschrittmachern vorhanden sind.

Die Polymerfolie ist vorzugsweise frei von Unterbrechungen und/oder durchgehenden Löchern oder Öffnungen. Dadurch lässt sich das medizinisch-technische Produkt vorteilhafterweise vollständig und insbesondere auch absolut dicht innerhalb der Polymerfolie einbetten.

Dadurch, dass das Polymer eine Polymermatrix bildet, in die die mindestens zwei antimikrobiellen Wirkstoffe homogen verteilt eingebettet sind, sind die Wirkstoffe überall im Bereich des Implantationsortes gleicherma-βen vorhanden. Es gibt vorzugsweise keine wirkstofffreien Bereiche, an denen es andernfalls zu Keimbildungen und Infektionen kommen könnte. Außerdem ist dadurch eine lange Verfügbarkeit der infektionshemmenden Wirkung sicher gestellt. Die mindestens zwei antimikrobiellen Wirkstoffe sind auch noch bei schon teilweise abgebautem Polymer vorhanden.

Dadurch, dass die Wirkstoffeinzelgehalte der mindestens zwei antimikrobiellen Wirkstoffe um höchstens 20 %, insbesondere um höchstens 10 %, voneinander abweichen, wird sichergestellt, dass die jeweils voneinander verschiedenen Wirkmechanismen der einzelnen Wirkstoffe praktisch gleichermaßen zur Geltung kommen und alle Bakterien und Keime im Wesentlichen im gleichen Umfang unterdrückt und/oder bekämpft werden.

Vorteilhafte Ausgestaltungen der erfindungsgemäßen Polymerfolie ergeben sich aus den Merkmalen der von Anspruch 1 abhängigen Ansprüche.

Günstig ist eine Ausgestaltung, bei der die Polymerfolie auf sich selbst haftet. Dadurch wird die Verbindbarkeit der Polymerfolie mit sich selbst verbessert. Weiterhin wird dadurch das in die Polymerfolie einzubettende medizinisch-technische Produkt sehr gut in der Polymerfolie gehalten. Außerdem verkleinern sich die Außenabmessungen der Einheit aus der zur Einbettung eingesetzten Polymerfolie und dem medizinisch-technischen Produkt, wenn die überstehenden Bereiche der Polymerfolie umgeklappt und auf einen anderen Bereich der Polymerfolie gelegt werden und in diesem umgeklappten Zustand aufgrund der vorteilhaften Selbsthaftung fixiert bleiben. Dies ermöglicht dem Arzt möglichst kleine Schnittöffnungen am Patientenkörper während der Operation zur Implantation des betreffenden medizinisch-technischen Produkts.

Gemäß einer weiteren günstigen Ausgestaltung ist die Polymerfolie mit sich selbst thermisch verschweißbar, insbesondere ohne Hilfsmittel und vorzugsweise klebstofffrei. Dadurch lassen sich aus der Polymerfolie auf einfache Weise andere Produkte, wie z.B. Taschen zur Aufnahme eines der zu implantierenden medizinisch-technischen Produkte, herstellen. Für die Verbindungsstelle oder -linie ist vorteilhafterweise kein anderer Stoff erforderlich, welcher andernfalls im Hinblick auf seine Bioverträglichkeit ausgewählt werden müsste. Dadurch sinkt der Aufwand. Außerdem bleibt die Biokompatibilität hoch.

Gemäß einer weiteren günstigen Ausgestaltung haftet die Polymerfolie auf einer metallischen Oberfläche. Zumindest einige der einzubettenden medizinisch-technischen Produkte haben ein metallisches Gehäuse, auf dem sich die Polymerfolie dann besonders gut und eng anschmiegt, vorzugsweise luftdicht und/oder glatt und insbesondere ohne, dass die Polymerfolie Blasen und/oder Wellen wirft. So werden Lufteinschlüsse vermieden, welche ansonsten unter dem Gesichtspunkt der Keimbildung problematisch sein könnten. Insbesondere besteht eine durchgängige Haftschicht zwischen der Polymerfolie und der betreffenden Metalloberfläche. Dies begünstigt eine möglichst stabile Sicherung des einzubettenden medizinisch-technischen Produkts in der Polymerfolie und verhindert Keimbewegungen vom oder zum medizinisch-technischen Produkt oder erschwert Letztere zumindest erheblich.

Gemäß einer weiteren günstigen Ausgestaltung ist die Polymerfolie eine physikalisch dichte Barriereschicht, die Bakterien und/oder Keine am Durchtritt hindert. Dadurch wird das Infektionsrisiko für den Patienten weiter reduziert.

Gemäß einer weiteren günstigen Ausgestaltung ist die Polymerfolie transparent. Dadurch erhält der Arzt eine unverstellte klare Sicht auf das Operationsgebiet und/oder das zu implantierende medizinisch-technische Produkt. Operationsfehler, die andernfalls dadurch geschehen können, dass ein hinter einer undurchsichtigen Folie verborgener Fremdkörper oder eine unzureichend behandelte Wundstelle übersehen werden, werden somit vermieden.

Gemäß einer weiteren günstigen Ausgestaltung sind die Wirkstoffeinzelgehalte der mindestens zwei antimikrobiellen Wirkstoffe zumindest etwa gleich groß. Insbesondere weichen die Wirkstoffeinzelgehalte also nicht voneinander ab. Dadurch wird sichergestellt, dass die jeweils voneinander verschiedenen Wirkmechanismen der einzelnen Wirkstoffe praktisch gleichermaßen zur Geltung kommen und alle Bakterien und Keime im Wesentlichen im gleichen Umfang unterdrückt und/oder bekämpft werden.

Gemäß einer weiteren günstigen Ausgestaltung hemmt ein erster antimikrobieller Wirkstoff die bakterielle Proteinbiosynthese und/oder die Nukleinsäuresynthese. Außerdem hemmt ein zweiter antimikrobieller Wirkstoff die bakterielle Zellwandsynthese. Dadurch werden möglichst viele der maßgeblichen Bakterien und Keime unterdrückt und/oder bekämpft. Beispiele für einen ersten antimikrobiellen Wirkstoff mit Hemmung der bakteriellen Proteinbiosynthese sind insbesondere Aminoglykoside (z.B. Amikacin, Gentamicin, Kanamycin, Neomycin, Netilmicin, Streptomycin, Tobramycin), Chloramphenicol, Fusidinsäure, Ketolide (z.B. Cethromycin, Narbomycin, Telithromycin), Lincosamide (z.B. Clindamycin, Lincomycin), Lipopeptide (z.B. Daptomycin), Streptogramine (z.B. Dalfopristin, Quinupristin), Makrolide (z.B. Azithromycin, Clarithromycin, Erythromycin, Roxithromycin), Oxazolidinone (z.B. Linezolid), Tetracycline (z.B. Doxycyclin, Minocyclin, Tetracyclin, Oxytetracyclin) und Glycylcycline (z.B. Tigecyclin). Beispiele für einen ersten antimikrobiellen Wirkstoff mit Hemmung der Nukleinsäuresynthese sind insbesondere Gyrasehemmer, also Hemmer der DNA-Replikation (wie Fluorchinolone der Generation 1 (z.B Norfloxacin, Enoxacin), Fluorchinolone der Generation 2 (z.B. Ciprofloxacin, Ofloxacin), Fluorchinolone der Generation 3 (z.B. Levofloxacin), Fluorchinolone der Generation 4 (z.B. Moxifloxacin), Aminocumarine), Hemmer der bakteriziden DNA-Schäden (wie Nitroimidazole (z.B. Metronidazol, Tinidazol, Nimorazol)), Folsäureantagonisten (wie Sulfonamide (z.B. Sulfadiazin, Sulfadoxin, Sulfamethoxazol, Sulfasalazin), Diaminopyrimidine (z.B. Pyrimethamin, Trimethoprim)) und Ansamycine, also Hemmer der bakteriellen RNA-Polymerase (wie Rifamycine (z.B. Rifampicin)). Beispiele für einen zweiten antimikrobiellen Wirkstoff mit Hemmung der bakteriellen Zellwandsynthese sind insbesondere β-Lactam-Antibiotika (wie Carbapeneme (z.B. Imipenem, Meropenem, Ertapenem), Cephalosporine, Monobactame (z.B. Aztreonam), Penicilline, wie Penicillin G, Penicillin V, Acylaminopenicilline (z.B. Piperacillin, Mezlocillin), Aminopenicilline (z.B. Ampicillin, Amoxicillin), Isoxazolylpenicilline (z.B. Flucloxacillin, Methicillin, Oxacillin), β-Lactamase-Hemmer (z.B. Clavulansäure, Sulbactam, Tazobactam) und Sultamicillin), aber auch Fosfomycin, Glycopeptide (z.B. Teicoplanin, Vancomycin), Polypeptide (z.B. Bacitracin, Colistin, Gramicidin, Polymyxin B, Tyrothricin, Teixobactin) und Fosmidomycin. Je nach Anwendungsfall können aber grundsätzlich auch noch andere antibakterielle Wirkstoffe zum Einsatz kommen.

Gemäß einer weiteren günstigen Ausgestaltung ist Gentamicin ein erster und Vancomycin ein zweiter der mindestens zwei antimikrobiellen Wirkstoffe, und hat die Polymerfolie einen Gentamicingehalt und einen Vancomycingehalt. Diese beiden Wirkstoffe lassen sich besonders gut mit jeweils hoher Konzentration in die Polymerfolie einbringen, ohne deren anderen vorteilhaften Eigenschaften, wie insbesondere die Biegbarkeit oder Biegefähigkeit, die Selbsthaftung, die thermische Verschweißbarkeit, die Haftung auf metallischen Oberflächen, die physikalische Unterbrechungsfreiheit, die Bildung einer physikalisch dichten Barriereschicht sowie die Transparenz, zu beeinträchtigen. Außerdem decken Gentamicin und Vancomycin praktisch das ganze Spektrum an Krankheitserregern und/oder Keimen ab, die für Infektionen bei der Implantation von medizinisch-technischen Produkten verantwortlich sind. Insbesondere decken diese beiden Wirkstoffe zumindest die folgenden wichtigen Krankheitserreger und/oder Keime ab: Coagulase (-) Staphylococcus, (z.B. Staphylococcus epidermidis), Staphylococcus aureus, Methicillin-sensitiver Staphylococcus aureus (MSSA), Methicillin-resistenter Staphylococcus aureus (MRSA), Escherichia coli, Haemophilus influenza und Moraxella catarrhalis, Enterococcus faecalis, Pseudomonas aeruginosa und Klebsiella pneumonia. Darüber hinaus sind Gentamicin und Vancomycin vorzugsweise sowohl unter physikalischen als auch unter mikrobiologischen Gesichtspunkten zueinander kompatibel. Es gibt insbesondere keine negative gegenseitige Beeinflussung der Wirkspektren. Der Wirkmechanismus des jeweils anderen Wirkstoffs bleibt unbeeinflusst. Weiterhin haben diese beiden Wirkstoffe eine sehr lange, insbesondere mehrjährige Lagerstabilität bzw. Haltbarkeit. Sowohl Gentamicin als auch Vancomycin lassen sich insbesondere jeweils mehr als 3 Jahre lagern, ohne dass es zu einer Verschlechterung der Wirkmechanismen kommt. Verglichen damit ist diese Haltbarkeit bei anderen Wirkstoffen deutlich kürzer, so beispielsweise bei Rifampicin wegen dessen Temperatur- und Lichtempfindlichkeit nur wenige Monate. Die Haltbarkeit bzw. Stabilität der Wirkstoffe bestimmt insbesondere auch die Haltbarkeit bzw. Stabilität sowie die Dauer der Verwendbarkeit der Polymerfolie an sich.

Gemäß einer weiteren günstigen Ausgestaltung beträgt das Verhältnis des Gentamicingehalts zu dem Polymergehalt und das Verhältnis des Vancomycingehalts zu dem Polymergehalt jeweils höchstens 32 %. Dann liegt für Gentamicin und für Vancomycin jeweils ein sehr hoher Wirkstoffeinzelgehalt mit sehr starker Keimunterdrückungswirkung vor, wobei aber immer noch die anderen oben genannten vorteilhaften Eigenschaften der Polymerfolie gegeben sind. Der Gentamicingehalt und der Vancomycingehalts können insbesondere jeweils als ein Flächengewicht des betreffenden der beiden Wirkstoffe angegeben werden. Insbesondere beträgt das Gentamicinflächengewicht und das Vancomycinflächengewicht jeweils höchstens 1,8 mg/cm², wobei das jeweilige Flächengewicht das Gewicht der Menge des betreffenden Wirkstoffs, die sich innerhalb eines cm² der Polymerfolie befindet, angibt. Insbesondere sind der Gentamicingehalt und der Vancomycingehalt zumindest in etwa gleich groß. Beide Wirkstoffe können somit ihre jeweilige Wirkung auf Krankheitserreger/Keime gleichermaßen entfalten. Der Polymergehalt ist insbesondere größer als die Summe des Gentamicingehalts und des Vancomycingehalts.

Gemäß einer weiteren günstigen Ausgestaltung beträgt das Verhältnis des Gentamicingehalts zu dem Polymergehalt und das Verhältnis des Vancomycingehalts zu dem Polymergehalt jeweils etwa 18 %. Vorzugsweise beträgt das Gentamicinflächengewicht und das Vancomycinflächengewicht jeweils etwa 1 mg/cm². Dann sind die Eigenschaften der Polymerfolie besonders gut.

Gemäß einer weiteren günstigen Ausgestaltung ist das biologisch abbaubare und vom menschlichen Körper resorbierbare Polymer natürlichen Ursprungs ein Polymer aus der Gruppe der Proteine, Polypeptide und Polysaccharide. Beispiele für Proteine sind insbesondere Elastin, Keratine und Kollagene. Beispiele für Polypeptide sind insbesondere Caseine, Gluten, Myosin und Kollagenhydrolysate wie z.B. Gelatine. Beispiele für Polysaccharide sind insbesondere Cellulose-Derivate, Oxicellulose, Viskose-Derivate, Cellulose-Acetate, Glycosaminglycane wie z.B. Hyaloronsäure, Stärke, Stärke-Derivate wie z.B. Hydroxyethylstärke, Chitosan, Lignin-Cellulose und Blends verschiedener Polymere.

Gemäß einer weiteren günstigen Ausgestaltung ist das biologisch abbaubare und vom menschlichen Körper resorbierbare Polymer natürlichen Ursprungs ein Atelokollagen equinen Ursprungs, und bevorzugt ein Typ 1 Atelokollagen equinen Ursprungs. Ein solches Polymer weist eine besonders hohe Biokompatibilität auf. Der menschliche Körper kann es besonders gut abbauen bzw. resorbieren. Das Kollagen equinen Ursprungs wird insbesondere aus Pferdesehnen gewonnen.

Gemäß einer weiteren günstigen Ausgestaltung hat die Polymerfolie ein Kollagenflächengewicht aus dem Bereich zwischen 2,5 mg/cm² und 8,0 mg/cm², insbesondere aus dem Bereich zwischen 5,0 mg/cm² und 6,2 mg/cm², und vorzugsweise von zumindest etwa 5,6 mg/cm² hat. Daraus resultiert eine Polymerfolie mit besonders guten Eigenschaften. Sie ist ausreichend stabil und reißfest für eine sichere Handhabung während der Operation, ohne einen zu hohen Materialeintrag in den Patienten zu bewirken. Außerdem ist sie gut biegbar und modulierbar. Das Kollagenflächengewicht ist dabei insbesondere eine Angabe für den Kollagengehalt der Polymerfolie.

Gemäß einer weiteren günstigen Ausgestaltung ist die Polymerfolie um mindestens 120° und vorzugsweise um bis zu 180° biegbar. Insbesondere wurden mit der Polymerfolie Biegetests mit Biegungen um etwa 170° durchgeführt, welche allesamt positiv, d.h. bruch- und zerstörungsfrei, verliefen. Eine so hohe Biegbarkeit ist ungewöhnlich. Sie verbessert die Handhabung und die Einsatzmöglichkeiten der Polymerfolie erheblich. Die Polymerfolie lässt sich insbesondere komplett Umbiegen oder Umklappen, also um 180° biegen, ohne dass es zu einem Bruch der Polymerfolie kommt. Der Biegevorgang kann auch mehrfach, insbesondere beliebig oft, ohne Bruch oder anderweitige Zerstörung der Polymerfolie wiederholt werden.

Eine weitere Aufgabe der Erfindung besteht darin, eine Polymertasche der eingangs bezeichneten Art mit gegenüber dem Stand der Technik verbesserten Eigenschaften anzugeben.

Zur Lösung der die Polymertasche betreffenden Aufgabe wird eine Polymertasche entsprechend den Merkmalen des Anspruchs 15 angegeben. Die erfindungsgemäße Polymertasche ist hergestellt aus mindestens einer erfindungsgemäßen Polymerfolie oder mindestens einer deren vorstehend beschriebenen günstigen Ausgestaltungen, wobei zwei aufeinander angeordnete Polymerfolien oder zwei aufeinander angeordnete Polymerfolien-Abschnitte randseitig bereichsweise miteinander verbunden sind, insbesondere thermisch verschweißt sind.

Vorteilhafterweise erfolgt diese randseitige Verbindung ohne Einsatz anderer Stoffe, insbesondere ohne einen Klebstoff.

Insbesondere resultiert je nach Ausgestaltung eine mechanisch feste Verbindungsstelle oder linienartige Verbindungszone. Es kann auch mehrere, z.B. zwei oder drei oder auch noch mehr, solcher Verbindungsstellen oder linienartigen Verbindungszonen geben. Darüber hinaus kann die Verbindung vorzugsweise auch (wasser- und/oder keim-)dicht sein.

Insbesondere kann die Polymertasche aus zwei übereinander platzierten Polymerfolien oder bevorzugt aus einer einzigen umgeklappten Polymerfolie jeweils mit entsprechender Verbindung in den maßgeblichen Randbereichen hergestellt sein. Die umgeklappte Ausgestaltung wird durch die gute Biegbarkeit der Polymerfolie begünstigt.

Die erfindungsgemäße Polymertasche ist ähnlich wie die erfindungsgemä-βe Polymerfolie zur Aufnahme eines in einen menschlichen Organismus zu implantierenden medizinisch-technischen Produkts bestimmt, insbesondere eines Herzschrittmachers, eines implantierbaren Defibrillators, eines Geräts zur Resynchronisationstherapie, einer implantierbaren Medikamentenpumpe, eines Neurostimulationsimplantats (Spinal Cord Stimulator, SCS), eines Kunstherzes, eines Blutdruck-regulierenden Implantats, eines Neuromodulators (Deep Brain Stimulation Device), aber auch eines mechanischen Implantats, einer Prothese oder dergleichen.

Ansonsten hat die erfindungsgemäße Polymertasche im Wesentlichen die gleichen günstigen Ausgestaltungen wie die vorstehend beschriebene Polymerfolie. Dabei bieten die erfindungsgemäße Polymertasche und ihre Ausgestaltungen im Wesentlichen die gleichen Vorteile, die bereits im Zusammenhang mit der erfindungsgemäßen Polymerfolie und deren Ausgestaltungen beschrieben worden sind.

Günstig ist eine weitere Ausgestaltung, bei der die Polymertasche eine Einführöffnung hat, in deren Bereich zwei sich einander gegenüberliegende Polymerfolien oder zwei sich einander gegenüberliegende Polymerfolien-Endabschnitte einer einzigen, insbesondere zur Bildung der Polymertasche umgeklappten, Polymerfolie unbündig enden, so dass im Bereich der Einführöffnung die eine Polymerfolie über die andere Polymerfolie oder der eine Polymerfolien-Endabschnitt über den anderen Polymerfolien-Endabschnitt hinaussteht. Der Überstand beträgt insbesondere 0,5 cm bis 1 cm. Er erleichtert das Einsetzen des zu implantierenden medizinisch-technischen Produkts.

Weitere Merkmale, Vorteile und Einzelheiten der Erfindung ergeben sich aus der nachfolgenden Beschreibung von Beispielen.
- Beispiel 1:: Herstellung einer Kollagenfolie (als Beispiel einer Polymerfolie) mit den beiden antimikrobiellen Wirkstoffen Gentamicin und Vancomycin

543 g Kollagen-Kuchen mit einem Trockengehalt an Typ 1 Atelokollagen equinen Ursprungs von 141 g werden zu 9000 ml gereinigtem, 40°C warmen Wasser, das zuvor mit verdünnter Essigsäure angesäuert worden ist, gegeben und mittels eines Homogenisators in eine homogene Suspension überführt. Es resultiert ein pH-Wert von 3,5 bis 4,2.

32 g Gentamicinsulfat werden in 500 ml angesäuertem Wasser gelöst und 21 g Vancomycinhydrochlorid werden in 500 ml angesäuertem Wasser gelöst.

Die Gentamicin-Lösung und die Vancomycin-Lösung werden so in die Kollagen-Suspension eingerührt, dass eine homogene Mischung aller drei Komponenten entsteht.

Diese homogene Mischung wird in flache Blisterformen mit einer Füllhöhe von ca. 5 mm abgefüllt.

Die Blisterformen werden in einen Klimaschrank verbracht, wo die homogene Mischung mit getrockneter, steriler Luft und einem Trocknungsregime mit Temperaturstufen von 35 °C bis 22 °C und einer Feuchte von 10-50% r.F. für 48 Stunden getrocknet wird.

Anschließend werden den Blisterformen die Kollagenfolien entnommen, die ein Kollagenflächengewicht von etwa 5,6 mg/cm², sowie ein Gentamicinflächengewicht und ein Vancomycinflächengewicht von jeweils etwa 1 mg/cm² haben. Diese Kollagenfolien sind transparent, haftfähig (sowohl auf sich selbst als auch auf metallischen Oberflächen) und biegbar, insbesondere flexibel bzw. geschmeidig und modulierbar. Die Wirkstoffe Gentamicin und Vancomycin sind homogen in der Kollagenfolie verteilt.
- Beispiel 2: Herstellung einer Kollagentasche (als Beispiel einer Polymertasche)

Jeweils zwei gemäß Beispiel 1 hergestellte Kollagenfolien werden in einem Klimaschrank bei 80% r.F. konditioniert, überlappend angeordnet und mittels einer Siegelmaschine dreiseitig zu einer Kollagentasche verpresst. Dabei herrscht ein Pressdruck von 8 bar und eine Temperatur von 100°C. Der Pressvorgang dauert eine halbe Minute. Während des Pressvorgangs erfolgt eine thermische Verschweißung der mit dem Pressdruck beaufschlagten drei Randbereiche. Die gepressten Bereiche sind linienartig. Dort resultieren Verbindungszonen, in denen zwischen den beiden Kollagenfolien eine mechanisch feste und dichte Verbindung besteht. Die linienartigen Verbindungszonen können auch als Siegelnähte bezeichnet werden. An einem Seitenrand ist keine Siegelnaht, sondern eine Einführöffnung vorhanden. Dort liegen die beiden Kollagenfolien nur aneinander an und sind insbesondere voneinander lösbar, beispielsweise, um ein zu implantierendes Gerät in die Kollagentasche einzusetzen.

Die verpresste Kollagentasche wird abschließend auf das Maß 9 x 9 cm zugeschnitten. Wegen die guten Biegbarkeit ist vorteilhafterweise nur eine Taschengröße erforderlich. Etwaige über das in die Kollagentasche eingesetzte zu implantierende Geräte seitlich überstehende leere Randbereiche der Kollagentasche können ohne weiteres umgeklappt werden. Die Größe der Kollagentasche lässt sich also problemlos an die Bedingungen des jeweiligen Anwendungsfalls anpassen.
- Beispiel 3: Prüfung der Siegelnaht-Belastbarkeit

In die Kollagentasche gemäß Beispiel 2 wird ein 24 g schwerer Herzschrittmacher-Dummy eingebracht. Die Stabilität der Kollagentasche und der Siegelnaht wird durch heftiges Schwenken und Schütteln der gefüllten Kollagentasche getestet. Sowohl die beiden Kollagenfolien der Kollagentasche als auch die die beiden Kollagenfolien bereichsweise verbindende Siegelnaht halten stand. Weder die Kollagenfolien noch die Siegelnaht platzen auf.

Die Kollagentasche gemäß Beispiel 2 wird außerdem mit 30 ml Wasser und damit zu etwa ¾ befüllt. Innerhalb von 30 min kommt es zu keinem Wasserverlust.
- Beispiel 4: Prüfung der Folienflexibilität - Biegetest

Kollagenfolien gemäß Beispiel 1 werden in Streifen mit einer Breite von 1,5 cm geschnitten. Jeweils ein solcher Streifen wird in zwei einander gegenüberstehende Klemmbackenpaare einer Zugprüfmaschine (Zwick 2.5) eingespannt, so dass der an seinen beiden Längsenden eingespannte Streifen zwischen den beiden Klemmbackenpaaren angeordnet ist. Der anfängliche Abstand der Klemmbackenpaare beträgt 10 mm. In diesem Zustand ist der von dem einen zu dem anderen Klemmbackenpaar verlaufende Streifen ungebogen.

Zur Prüfung wird das erste Klemmbackenpaar mit einer Geschwindigkeit von 300 mm/min auf das zweite Klemmbackenpaar zu bewegt, bis sie nur noch einen Abstand von 1 mm voneinander haben. Danach wird das erste Klemmbackenpaar wieder in seine Ausgangsposition zurück bewegt. Dieser Vorgang wird 20 Mal wiederholt. Während der Bewegung des ersten Klemmbackpaares vollzieht der beidseitig eingespannte Folienstreifen eine mehrfache Biege-Ausweichbewegung zur Seite, wobei der Folienstreifen sich umso stärker verbiegt, je näher das erste Klemmbackenpaar dem zweiten Klemmbackenpaar kommt.

Während dieser Biegeprüfung bricht keiner der getesteten Streifen. Die Kollagenfolie ist also sehr geschmeidig. Sie lässt sich ohne Beschädigung stark verbiegen. Während der Biegeprüfung erfolgten Verbiegungen um bis zu etwa 170°.

Bei Vergleichsfolien anderer Zusammensetzung ist es dagegen während der Biegeprüfung zum Bruch der getesteten Streifen gekommen.
- Beispiel 5: Prüfung der Haftfähigkeit

Kollagenfolien gemäß Beispiel 1 werden in Streifen mit einer Breite von 1,5 cm und einer Länge von mehr als 5 cm geschnitten. Ein Teilbereich von 2 cm Länge dient jeweils als zu testende Haftfläche, welche auch markiert wird.

Pro Test werden je zwei der Folienstreifen aufeinandergelegt, kurz in den markierten Teilbereichen mit den zu testenden und aufeinander platzierten Haftflächen angefeuchtet (da die Befeuchtung die ohnehin bereits gute Haftung noch einmal deutlich erhöht und damit auch besser messbar macht) und nach verschiedenen Verweilzeiten (3-22 min) mittels der schon bei den Prüfungen gemäß Beispiel 4 eingesetzten Zugprüfmaschine (Zwick 2.5) auseinander gezogen.

Obwohl die Haftkraft mit längerer Verweildauer zunimmt, wird für die weiteren vergleichenden Untersuchungen eine Verweilzeit von 10 min gewählt, da einerseits die Messgenauigkeit dann schon ausreichend gut ist und anderseits auch bei einer realen Implantations-Operation eine Verweil- bzw. Vorbereitungszeit von maximal 10 min bis zum Einführen des zu implantierenden Geräts, beispielsweise eines Herzschrittmachers, in die Gewebetasche des Patienten realistisch ist.

Bei den aus den Kollagenfolien gemäß dem Beispiel 1 gewonnen Streifen liegt die mittlere gemessene Haftkraft bei 625 mN, was ein sehr guter Wert ist, der nahe an der vergleichbaren Haftkraft einer reinen Kollagenfolie ohne jeglichen antimikrobiellen Wirkstoff liegt. Bei Vergleichsfolien von Produkten anderer Hersteller, die ebenfalls zur Einbettung von zu implantierenden Herzschrittmachern dienen, liegt die mittlere gemessene Haftkraft demgegenüber deutlich niedriger. Eine Vergleichsfolie hat eine so ermittelte Haftkraft von nur 20 mN. Bei einer anderen Vergleichsfolie ergibt sich sogar überhaupt keine Haftkraft. Der ermittelte Wert liegt bei 0 mN.
- Beispiel 6: Prüfung der antibiotischen Wirkung

Die mikrobiologische Aktivität der Kollagenfolie gemäß Beispiel 1 wird in Gegenwart von 3 Gram-positiven und 3 Gram-negativen Keimen getestet. Als Keime werden verwendet Staphylococcus epidermidis, Staphylococcus aureus, Enterococcus faecalis, Escherichia coli, Pseudomonas aeruginosa und Klebsiella pneumonia. Außerdem wird die mikrobielle Wirkung gegen einen Gentamicin-resistenten Keim (Staph aureus, >16mg/ml) getestet.

Für die Prüfungen werden jeweils 3 Stücke á 3x3 cm der Kollagenfolie für 24 Stunden mit 2,5 x 10⁶ CFU/ inoculum bei 37 °C gemäß ASTM E 2180 im Vergleich zu nicht mikrobiell beladenen Kollagenfolien inkubiert.

Die Kollagenfolie gemäß Beispiel 1 weist gegen alle getesteten Keime eine Reduktion von mehr als 4 logarithmischen Stufen auf. Gemäß den einschlägigen Bestimmungen gilt ein antimikrobieller Test als bestanden, wenn eine Keimreduktion von mindestens 99,9 % (also mindestens 3 logarithmischen Stufen) im Vergleich zur Referenz erreicht wird. Dieses Bewertungskriterium ist im vorliegenden Fall erfüllt. Die Kollagenfolie hat somit den antimikrobiellen Test bestanden.

## Patentansprüche

1. Polymerfolie zur Einbettung eines in einen menschlichen Organismus zu implantierenden medizinisch-technischen Produkts, wobei
a) die Polymerfolie als Hauptbestandteil ein biologisch abbaubares und vom menschlichen Körper resorbierbares Polymer natürlichen Ursprungs, nämlich ein Atelokollagen, aufweist und die Polymerfolie einen Polymergehalt hat,
b) die Polymerfolie als weitere Bestandteile mindestens zwei antimikrobielle Wirkstoffe mit jeweils unterschiedlichem Wirkmechanismus aufweist und die Polymerfolie einen Wirkstoffgesamtgehalt hat,
c) wobei das Verhältnis des Wirkstoffgesamtgehalts zu dem Polymergehalt mindestens 15 % beträgt, der Polymergehalt aber größer als der Wirkstoffgesamtgehalt ist,
d) die Polymerfolie biegbar und modulierbar ist, so dass die Polymerfolie an das medizinisch-technische Produkt eng angelegt werden kann, wobei sich die Polymerfolie zumindest weitestgehend an die Konturen des medizinisch-technischen Produkts anpasst,
e) die Polymerfolie ununterbrochen ist,
f) das Polymer eine Polymermatrix bildet, in die die mindestens zwei antimikrobiellen Wirkstoffe homogen verteilt eingebettet sind, und
g) die Polymerfolie für jeden der mindestens zwei antimikrobiellen Wirkstoffe jeweils einen Wirkstoffeinzelgehalt hat, wobei die Wirkstoffeinzelgehalte um höchstens 20 % voneinander abweichen.

2. Polymerfolie nach Anspruch 1, **dadurch gekennzeichnet, dass** sie auf sich selbst haftet.

3. Polymerfolie nach Anspruch 1 oder 2, **dadurch gekennzeichnet, dass** sie mit sich selbst thermisch verschweißbar ist.

4. Polymerfolie nach einem der vorhergehenden Ansprüche, **dadurch gekennzeichnet, dass** sie auf einer metallischen Oberfläche haftet.

5. Polymerfolie nach einem der vorhergehenden Ansprüche, **dadurch gekennzeichnet, dass** sie eine physikalisch dichte Barriereschicht ist, die Bakterien am Durchtritt hindert.

6. Polymerfolie nach einem der vorhergehenden Ansprüche, **dadurch gekennzeichnet, dass** sie transparent ist.

7. Polymerfolie nach einem der vorhergehenden Ansprüche, **dadurch gekennzeichnet, dass** die Wirkstoffeinzelgehalte der mindestens zwei antimikrobiellen Wirkstoffe zumindest etwa gleich groß sind.

8. Polymerfolie nach einem der vorhergehenden Ansprüche, **dadurch gekennzeichnet, dass** ein erster antimikrobieller Wirkstoff die bakterielle Proteinbiosynthese und/oder die Nukleinsäuresynthese hemmt sowie ein zweiter antimikrobieller Wirkstoff die bakterielle Zellwandsynthese hemmt.

9. Polymerfolie nach einem der vorhergehenden Ansprüche, **dadurch gekennzeichnet, dass** Gentamicin ein erster und Vancomycin ein zweiter der mindestens zwei antimikrobiellen Wirkstoffe ist, und die Polymerfolie einen Gentamicingehalt und einen Vancomycingehalt hat.

10. Polymerfolie nach Anspruch 9, **dadurch gekennzeichnet, dass** das Verhältnis des Gentamicingehalts zu dem Polymergehalt und das Verhältnis des Vancomycingehalts zu dem Polymergehalt jeweils höchstens 32 % beträgt.

11. Polymerfolie nach Anspruch 10, **dadurch gekennzeichnet, dass** das Verhältnis des Gentamicingehalts zu dem Polymergehalt und das Verhältnis des Vancomycingehalts zu dem Polymergehalt jeweils etwa 18 % beträgt.

12. Polymerfolie nach einem der vorhergehenden Ansprüche, **dadurch gekennzeichnet, dass** das biologisch abbaubare und vom menschlichen Körper resorbierbare Polymer natürlichen Ursprungs ein Atelokollagen equinen Ursprungs ist, und bevorzugt ein Typ 1 Atelokollagen equinen Ursprungs.

13. Polymerfolie nach einem der vorhergehenden Ansprüche, **dadurch gekennzeichnet, dass** die Polymerfolie ein Kollagenflächengewicht aus dem Bereich zwischen 2,5 mg/cm² und 8,0 mg/cm², insbesondere aus dem Bereich zwischen 5,0 mg/cm² und 6,2 mg/cm², und vorzugsweise von zumindest etwa 5,6 mg/cm² hat.

14. Polymerfolie nach einem der vorhergehenden Ansprüche, **dadurch gekennzeichnet, dass** die Polymerfolie um mindestens 120° und vorzugsweise um bis zu 180° biegbar ist.

15. Polymertasche zur Aufnahme eines in einen menschlichen Organismus zu implantierenden medizinisch-technischen Produkts, hergestellt aus mindestens einer Polymerfolie nach einem der vorhergehenden Ansprüche, wobei zwei aufeinander angeordnete Polymerfolien oder zwei aufeinander angeordnete Polymerfolien-Abschnitte randseitig bereichsweise miteinander verbunden sind.

16. Polymertasche nach Anspruch 15, **dadurch gekennzeichnet, dass** eine Einführöffnung vorhanden ist, in deren Bereich zwei sich einander gegenüberliegende Polymerfolien oder zwei sich einander gegenüberliegende Polymerfolien-Endabschnitte einer einzigen Polymerfolie unbündig enden, so dass im Bereich der Einführöffnung die eine Polymerfolie über die andere Polymerfolie oder der eine Polymerfolien-Endabschnitt über den anderen Polymerfolien-Endabschnitt hinaussteht.

## Claims

1. Polymer film for the embedding of a medical technology product to be implanted in a human organism, wherein
a) the polymer film comprises, as main constituent, a polymer of natural origin that is biodegradable, namely an atelocollagen, which is absorbable by the human body, and the polymer film has a polymer content,
b) the polymer film comprises, as further constituents, at least two antimicrobial active ingredients having a different mechanism of action in each case and the polymer film has a total active-ingredient content,
c) wherein the ratio of the total active-ingredient content to the polymer content is at least 15 %, but the polymer content is greater than the total active-ingredient content,
d) the polymer film is bendable and modulable, so that the polymer film can be applied closely to the medical technology product, wherein the polymer film at least mostly matches itself to the contours of the medical technology product,
e) the polymer film is uninterrupted,
f) the polymer forms a polymer matrix in which the at least two antimicrobial active ingredients are embedded in a homogeneously distributed manner, and
g) the polymer film has, for each of the at least two antimicrobial active ingredients, an individual active-ingredient content in each case, the individual active-ingredient contents deviating from one another by at most 20 %.

2. Polymer film according to claim 1, **characterized in that** it adheres to itself.

3. Polymer film according to claim 1 or 2, **characterized in that** it is heat-sealable with itself.

4. Polymer film according to any one of the preceding claims, **characterized in that** it adheres to a metallic surface.

5. Polymer film according to any one of the preceding claims, **characterized in that** it is a physically tight barrier layer which prevents bacteria from passing through.

6. Polymer film according to any one of the preceding claims, **characterized in that** it is transparent.

7. Polymer film according to any one of the preceding claims, **characterized in that** the individual active-ingredient contents of the at least two antimicrobial active ingredients are at least about the same size.

8. Polymer film according to any one of the preceding claims, **characterized in that** a first antimicrobial active ingredient inhibits the bacterial biosynthesis of proteins and/or synthesis of nucleic acids and a second antimicrobial active ingredient inhibits the bacterial synthesis of the cell wall.

9. Polymer film according to any one of the preceding claims, **characterized in that** gentamicin is a first antimicrobial active ingredient and vancomycin is a second antimicrobial active ingredient of the at least two antimicrobial ingredients, and the polymer film has a gentamicin content and a vancomycin content.

10. Polymer film according to claim 9, **characterized in that** the ratio of the gentamicin content to the polymer content and the ratio of the vancomycin content to the polymer content is at most 32% in both cases.

11. Polymer film according to claim 10, **characterized in that** the ratio of the gentamicin content to the polymer content and the ratio of the vancomycin content to the polymer content is about 18% in both cases.

12. Polymer film according to any one of the preceding claims, **characterized in that** the polymer of natural origin that is biodegradable and absorbable by the human body is an atelocollagen of equine origin, and preferably a type 1 atelocollagen of equine origin.

13. Polymer film according to any one of the preceding claims, **characterized in that** the polymer film has a collagen basis weight from the range between 2.5 mg/cm² and 8.0 mg/cm² , in particular from the range between 5.0 mg/cm² and 6.2 mg/cm² , and preferably of at least about 5.6 mg/cm² .

14. Polymer film according to any one of the preceding claims, **characterized in that** the polymer film is bendable by at least 120° and preferably by up to 180°.

15. Polymer pouch for receiving a medical technology product to be implanted in a human organism, produced from at least one polymer film according to any one of the preceding claims, with two polymer films arranged on top of one another being regionally connected to one another on the edge side or two polymer-film sections arranged on top of one another being regionally connected to one another on the edge side.

16. Polymer pouch according to claim 15, **characterized in that** there is an introduction opening, in the region of which two opposing polymer films or two opposing polymer-film end sections of a single polymer film end in a non-flush manner, with the result that, in the region of the introduction opening, one polymer film projects beyond the other polymer film or one polymer-film end section projects beyond the other polymer-film end section.

## Revendications

1. Film polymère destiné à incorporer un produit médico-technique à implanter dans un organisme humain, dans lequel
a) le film polymère présente comme composant principal un polymère d'origine naturelle biodégradable et résorbable par le corps humain, à savoir un atélocollagène, et le film polymère a une teneur en polymère,
b) le film polymère présente comme autres composants au moins deux substances actives antimicrobiennes ayant chacune un mécanisme d'action différent et le film polymère a une teneur totale en substance active,
c) le rapport entre la teneur totale en substance active et la teneur en polymère étant d'au moins 15 %, mais la teneur en polymère étant supérieure à la teneur totale en substance active,
d) le film polymère est pliable et modulable, de sorte que le film polymère peut être appliqué étroitement contre le produit médico-technique, le film polymère s'adaptant au moins dans une large mesure aux contours du produit médico-technique,
e) le film polymère est ininterrompu,
f) le polymère forme une matrice polymère dans laquelle les au moins deux substances actives antimicrobiennes sont incorporées et réparties de manière homogène, et
g) le film polymère a une teneur individuelle en substance active pour chacune des au moins deux substances actives antimicrobiennes, les teneurs individuelles en substance active ne différant pas de plus de 20 % les unes des autres.

2. Film polymère selon la revendication 1, **caractérisé en ce qu'**il adhère sur lui-même.

3. Film polymère selon la revendication 1 ou 2, **caractérisé en ce qu'**il est thermosoudable sur lui-même.

4. Film polymère selon l'une quelconque des revendications précédentes, **caractérisé en ce qu'**il adhère à une surface métallique.

5. Film polymère selon l'une quelconque des revendications précédentes, **caractérisé en ce qu'**il est une couche barrière physiquement étanche qui empêche le passage des bactéries.

6. Film polymère selon l'une quelconque des revendications précédentes, **caractérisé en ce qu'**il est transparent.

7. Film polymère selon l'une quelconque des revendications précédentes, **caractérisé en ce que** les teneurs individuelles en substances actives des au moins deux substances actives antimicrobiennes sont au moins approximativement égales.

8. Film polymère selon l'une quelconque des revendications précédentes, **caractérisé en ce qu'**une première substance active antimicrobienne inhibe la biosynthèse des protéines bactériennes et/ou la synthèse des acides nucléiques, ainsi qu'une deuxième substance active antimicrobienne inhibe la synthèse de la paroi cellulaire bactérienne.

9. Film polymère selon l'une quelconque des revendications précédentes, **caractérisé en ce que** la gentamicine est une première substance active antimicrobienne et la vancomycine est une deuxième substance active antimicrobienne parmi les au moins deux substances actives antimicrobiennes, et le film polymère a une teneur en gentamicine et une teneur en vancomycine.

10. Film polymère selon la revendication 9, **caractérisé en ce que** le rapport de la teneur en gentamicine à la teneur en polymère et le rapport de la teneur en vancomycine à la teneur en polymère sont chacun d'au plus 32 %.

11. Film polymère selon la revendication 10, **caractérisé en ce que** le rapport de la teneur en gentamicine à la teneur en polymère et le rapport de la teneur en vancomycine à la teneur en polymère sont chacun d'environ 18 %.

12. Film polymère selon l'une quelconque des revendications précédentes, **caractérisé en ce que** le polymère d'origine naturelle biodégradable et résorbable par le corps humain est un atélocollagène d'origine équine, et de préférence un atélocollagène de type 1 d'origine équine.

13. Film polymère selon l'une quelconque des revendications précédentes, **caractérisé en ce que** le film polymère a un grammage de collagène compris entre 2,5 mg/cm² et 8,0 mg/cm², en particulier entre 5,0 mg/cm² et 6,2 mg/cm², et de préférence d'au moins environ 5,6 mg/cm².

14. Film polymère selon l'une quelconque des revendications précédentes, **caractérisé en ce que** le film polymère peut être plié d'au moins 120° et de préférence jusqu'à 180°.

15. Poche polymère destinée à recevoir un produit médico-technique à implanter dans un organisme humain, fabriquée à partir d'au moins un film polymère selon l'une quelconque des revendications précédentes, dans laquelle deux films polymères superposés ou deux sections de films polymères superposées sont reliées entre elles par zones sur les bords.

16. Poche polymère selon la revendication 15, **caractérisée en ce qu'**il existe une ouverture d'introduction dans la zone de laquelle deux films polymères opposés l'un à l'autre ou deux sections d'extrémité de film polymère opposées l'une à l'autre d'un seul film polymère se terminent sans affleurement, de sorte que dans la zone de l'ouverture d'introduction, un film polymère dépasse l'autre film polymère ou l'une des sections d'extrémité de film polymère dépasse l'autre section d'extrémité de film polymère.
